(19) Europäisches Patentamt
European Patent Office
Office européen des brevets

(11) Publication number: **0 082 873**
**B1**

(12) # EUROPEAN PATENT SPECIFICATION

(45) Date of publication of patent specification: **07.12.88**

(51) Int. Cl.⁴: **A 61 K 31/24,** A 61 K 31/60, A 61 K 31/245, A 61 K 31/275

(21) Application number: **82902333.2**

(22) Date of filing: **23.06.82**

(86) International application number: **PCT/US82/00849**

(87) International publication number: **WO 83/00014 06.01.83 Gazette 83/01**

(54) **COMPOSITIONS FOR TREATING GLAUCOMA.**

(30) Priority: **23.06.81 US 276658**

(43) Date of publication of application: **06.07.83 Bulletin 83/27**

(45) Publication of the grant of the patent: **07.12.88 Bulletin 88/49**

(84) Designated Contracting States: **BE FR**

(56) References cited:
**EP-A-0 003 664**
**EP-A-0 041 491**
**EP-A-0 053 435**
**FR-A-1 566 349**
**FR-A-2 110 230**
**FR-A-2 132 570**
**US-A-3 663 607**
**US-A-3 740 444**
**US-A-3 793 365**
**US-A-3 825 583**
**US-A-3 839 584**
**US-A-3 868 460**
**US-A-3 925 446**
**US-A-4 080 471**
**US-A-4 146 638**
**US-A-4 191 765**

(73) Proprietor: **AMERICAN HOSPITAL SUPPLY CORPORATION**
**One American Plaza**
**Evanston, IL 60201 (US)**

(72) Inventor: **MATIER, William Lesley**
**1214 Parliament Court**
**Libertyville, IL 60048 (US)**

(74) Representative: **Maiffret, Bernard**
**Law Offices of William J. Rezac 49, avenue Franklin D. Roosevelt**
**F-75008 Paris (FR)**

(56) References cited:
**CHEMICAL ABSTRACTS, vol. 80, no. 17 April 29, 1974, page 377, abstract no. 95546w COLUMBUS OHIO (US)**

**N, Glaucoma, Issued 1980, Bonomi et al Beta-adrenergic Blocking agents and Intraocular pressure: Comparative Evaluation of Twelve Drugs, See Pages 99-107**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 082 873 B1**

(56) References cited:
**N, Investigative Ophthalmology and Visual Science,Volume 16, No. 11, issued November 1977, Vareilles et al., Comparison of the effects of timolol and other adrenergic agents on intraocular pressure in the rabbit, See pages 987-996.**

**N, Survey of Ophthalmology, Volume 23, No. 6 issued May-June 1979, Zimmerman et al., The Beta-Adrenergic Blocking Agents and the treatment of Glaucoma, See Pages 347-362.**

**Description**

The present invention relates to the use of a compound for the manufacture of a medicament for the treatment of glaucoma, or lowering of intraocular pressure.

Glaucoma is a condition of the eye characterised by increased intraocular pressure. Untreated, the condition can eventually lead to irreversible retinal damage and blindness. Conventional therapy for glaucoma has involved topical administration of pilocarpine and/or epinephrine, administered to the eye several times daily.

Various *beta*-blocking agents may also be used to lower intraocular pressure. Such use is described, for example, in reviews by W. P. Boger in *Drugs, 18,* 25—32 (1979) and by T. J. Zimmerman & W. P. Boger in *Surv. Ophthalaol, 23(c),* 347 (1979). The use of *beta*-blockers for the treatment of glaucoma is also described in the patent literature. For example, U.S. Patent No. 4,195,085 to Stone discloses a method for treatment of glaucoma by the ocular administration of a *beta*-blocking compound, timolol maleate. U.S. Patent No. 4,127,674 discloses treating glaucoma with labetalol, a known antagonist of both alpha and beta adrenergic receptors. However, these methods also possess significant drawbacks, in that the absorption of the *beta*-blocking compound into the systemic circulation can cause undesirable side effects. Such side effects result from prolonged *beta*-blocking action on the heart, bronchioles and blood vessels. For example, according to *Physicians' Desk Reference*, Charles E. Baker, Jr., 35th Edition, 1981, p. 1233, adverse reactions to the topical use of timolol maleate can include bronchospasm, heart failure, as well as cardiac conduction defects. Accordingly, there is a need for a method of treatment for glaucoma or for lowering intraocular pressure utilizing *beta*-blocking agents which are relatively free of unwanted systemic side-effects.

Certain *beta*-blocking agents which contain enzymatically labile ester groups are known to exhibit short-acting *beta*-blocking effects in the systemic circulation. Such short-acting *beta*-blocking compounds (SABBs) have been suggested for treatment or prophylaxis of cardiac disorders as a means for reducing heart work or improving rhythmicity for a short duration. Such short-acting *beta*-blocking compounds avoid the sometimes counterproductive effects of conventional *beta*-blocking agents, whose effects are typically long-lived and, therefore, difficult to precisely control.

The present invention relates to the use of a compound of the formula

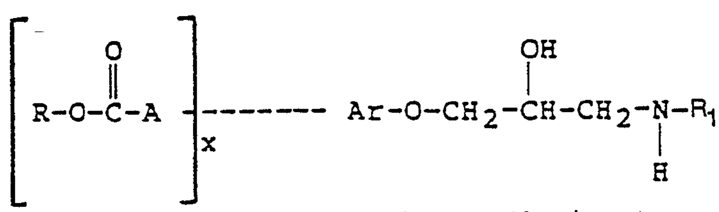

wherein R is alkyl of straight or branched carbon chains from 1 to 10 carbon atoms, cycloalkyl of from 3 to 5 carbon atoms, alkenyl of from 2 to 5 carbon atoms, alkynyl of from 2 to 5 carbon atoms, alkyl carboxymethyl in which the alkyl portion contains from 1 to 5 carbon atoms, aryl carboxymethyl in which the aryl portion contains from 6 to 8 carbon atoms, aryl of from 6 to 10 carbon atoms, or aralkyl wherein the alkyl portion contains from 1 to 5 carbon atoms and the aryl portion represents substituted or unsubstituted monocyclic or polycyclic aromatic of from 6 to 10 carbon atoms; A is a direct bond, alkylkene of from 1 to 5 carbon atoms, or alkylene of from 1 to 5 carbon atoms, or alkenylene of from 2 to 5 carbon atoms; x is an integer from 1 to 3, provided that when x is greater than 1, different occurrences of the

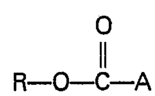

group may be the same or different; Ar is phenyl or naphthyl optionally additionally substituted with $C_1$—$C_{10}$ alkyl, $C_2$—$C_5$ alkenyl, $C_2$—$C_5$ alkynyl, halogen, acetamido, amino, nitro, ($C_1$—$C_{10}$ alkyl) amino, hydroxy, $C_1$—$C_{10}$ hydroxyalkyl, or cyano; $R_1$ is $C_1$—$C_{10}$ alkyl, $C_1$—$C_{10}$ hydroxyalkyl, $C_2$—$C_5$ alkenyl, $C_2$—$C_5$ alkynyl or aralkyl; or a pharmaceutically acceptable salt thereof, for the manufacture of a medicament for the treatment of glaucoma or lowering of intraocular pressure.

The above-mentioned short-acting *beta*-blocking compounds effectively reduce intraocular pressure in the eyes of mammals when topically administered. Because of their short-lived duration of action in the systemic circulation, side-effects produced by their migration out of the eye are consequently reduced. It has further been discovered that certain of these compounds show an increased longevity of effect when present in the ocular fluid, compared to the duration of their systemic effects. Consequently, the present invention resides in the treatment of glaucoma or lowering intraocular pressure with a *beta*-blocking compound which exhibits relatively long duration of action while in the ocular fluid, but which is subject to relatively rapid breakdown into inactive metabolites upon passage to the systemic circulation.

The compounds may be administered as their pharmaceutically acceptable acid addition salts, e.g., as the hydrochloride, sulfate, phosphate, gluconate, and tartrate.

In preferred compounds, A is a direct bond, alkylene of from 1 to 5 carbon atoms, such as methylene, ethylene, butylene, or alkenyl of from 2 to 5 carbon atoms, such as ethenyl, 2-propenyl, 2-butenyl, and x is 1 or 2, and in particularly preferred compounds, Ar is phenyl, x is 1 or 2 and A is a direct bond, alkylene of from 1 to 3 carbon atoms, or alkenyl of 2 carbon atoms. It has been found that the compounds in which Ar is phenyl and para-substituted, *beta*-blocking potency and shortness of duration of action in blood are improved when A is alkylene, i.e., the ester group is isolated from the aromatic ring by at least one methylene unit. Alternatively, in compounds in which Ar is phenyl, at least one of the ester-containing groups,

$$R-O-\overset{O}{\overset{\|}{C}}-,$$

is advantageously in the ortho-position with respect to the side chain. It is surprising and presently unexplained, that two configurations of the compounds of the present invention, para-substitution with an ester carbonyl isolated from the aromatic ring and ortho-substitution with the ester carbonyl attached directly to the aromatic ring, provide enhanced *beta*-blocking potency and relatively short duration of action in blood.

The ester substituent, R, in preferred compounds, is alkyl of from 1 to about 5 carbon atoms, such as mthyl, ethyl, n-butyl, n-pentyl; alkenyl of from 2 to 5 carbon atoms, such as ethenyl, 2-propenyl, 2-methyl-3-butenyl, alkynyl of from 3 to 5 carbon atoms, such as propargyl, methylpropargyl, or cycloalkyl of from 3 to 5 carbon atoms such as cyclopropyl, cyclopentyl, 2-methylcyclopropyl; $R_1$ is preferably alkyl of from 1 to about 5 carbon atoms such as methyl, ethyl, propyl, t-butyl, pentyl, hydroxyalkyl of from 1 to 5 carbon atoms, such as hydroxyethyl, hydroxy-t-butyl, hydroxyisopropyl; alkynyl of from 3 to 5 carbon atoms such as propargyl, dimethylpropargyl; or aralkyl, wherein the alkyl portion contains from 1 to 5 carbon atoms and the aryl portion contains from 6 to 10 carbon atoms, such as benzyl, phenethyl, dimethoxyphenethyl, naphthylethyl, phenylbutyl.

Preferred aromatic substituents include alkyl of from 1 to 5 carbon atoms, alkenyl of from 2 to 5 carbon atoms, alkoxy of from 1 to 5 carbon atoms, halogen, acetamido, amino, nitro, alkylamino of from 1 to 5 carbon atoms, hydroxy, hydroxyalkyl of from 1 to 5 carbon atoms, and cyano. Particularly preferred aromatic substituents are alkyl of from 1 to 5 carbon atoms, fluoro, chloro, cyano, and alkoxy.

The compounds described herein, as well as their methods of preparation, are disclosed in co-pending U.S. Patent Application, Serial No. 211,345, corresponding to EP—A—53435. In addition, certain of the compounds which may be employed in the method of the present invention are known in the art. For instance, compounds of the above formula in which A is ethenyl are described in U.S. Patent 4,191,765 and in Japanese unexamined patent application (Kokai) 7400247 (see also *Chemical Abstracts, 80,* 95546W (1974)). Compounds of the above formula in which $R_1$ is 1,1-dimethyl-2-hydroxyethyl are described in British Patent 1,364,280, and compounds in which $R_1$ is dimethylpropargyl are described in British Patent 1,450,287.

The compounds are advantageously administered topically to the eye in the form of a solution, ointment, or solid insert such as is described in U.S. Patent No. 4,195,085 to allow controlled or delayed release of the drug. Formulations may contain the active compound, preferably, in the form of a soluble acid addition salt, in amounts ranging from about 0.01 to about 10% by wt., preferably, from about 0.5% to about 5% by wt. Unit dosages of the active compound can range from about 0.001 to about 5.0 mg, preferably from about 0.05 to about 2.0 mg. The dosage administered to a patient will depend upon the patient's needs and the particular compounds employed.

Carriers used in the preparations of the present invention are preferably non-toxic pharmaceutical organic or inorganic compositions such as water; mixtures of water and water-miscible solvents, such as lower alcohols; mineral oils; petroleum jellies; ethyl cellulose; polyvinylpyrrolidone and other conventional carriers. In addition, the pharmaceutical preparations may also contain additional components such as emulsifying, preserving, wetting and sterilizing agents. These include polyethylene glycols 200, 300, 400 and 600, carbowaxes 1,000, 1,500, 4,000, 6,000 and 10,000, bacteriocidal components such as quaternary ammonium compounds, phenylmercuric salts known to have cold sterilizng properties and which are non-injurious in use, thimerosal, methyl and propyl paraben, benzyl alcohol, phenyl ethanol, buffering ingredients such as sodium chloride, sodium borate, sodium acetates, gluconate buffers, and other conventional ingredients such as sorbitan monolaurate, triethanolamine, oleate, polyoxyethylene sorbitan monopalmitylate, dioctyl sodium sulfosuccinate, monothioglycerol, thiosorbitol and ethylenediamine tetracetic acid. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like.

Formulations for eye drops preferably include the active compounds as a soluble acid addition salt in a properly buffered, sterile, aqueous isotonic solution.

The compounds employed in the use of the present invention are ester group-containing *beta*-blockers that have a selective, localized, *beta*-blocking effect in the eye after topical administration. Such compounds are thought to be rapidly metabolized by plasma and/or liver esterases into inactive byproducts, upon entering the systemic circulation. It has been discovered that these same compounds are relatively stable in ocular fluids, i.e., lacrimal fluids and aqueous humor. Consequently, such compounds are useful for the treatment of glaucoma or for lowering intraocular pressure since they remain stable when topically applied to the eye but rapidly metabolize when subsequently absorbed into the systemic circulation.

Some of the compounds break down in the aqueous humor more rapidly than others. Such compounds may advantageously be employed when only a temporary reduction in intraocular pressure is desired, say for diagnostic procedures. Longer-acting compounds are generally used for effecting longer-term reductions in intraocular pressure, such as is desired when treating chronic glaucoma. Thus, the compounds provide a very useful therapeutic alternative for the treatment of glaucoma or for lowering intraocular pressure.

The *in vitro* studies hereinafter described indicate that the compounds used in the present invention will undergo different rates of enzymatic hydrolysis depending on their location within the body (see Table I). For example, the compound of Example I is completely hydrolyzed within 60 minutes in liver homogenate while only 0.3% hydrolyzed after one hour in aqueous humor, and only 1.3% hydrolyzed after two hours. The compound of Example V is less stable in aqueous humor, hydrolyzing 3.6% after one hour, 13.4% after two hours.

The present invention is further illustrated by the following examples.

Examples I—V

The enzymatic hydrolysis rates of the following compounds were examined in dog blood, liver homogenate, and aqueous humor:

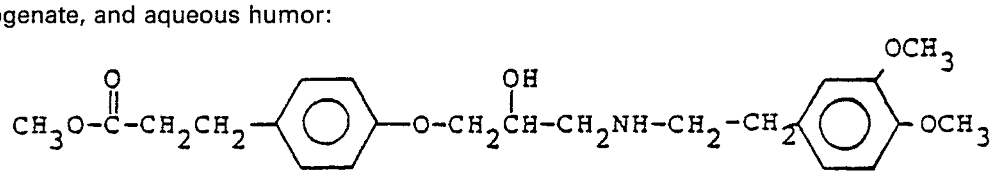

COMPOUND OF EXAMPLE I

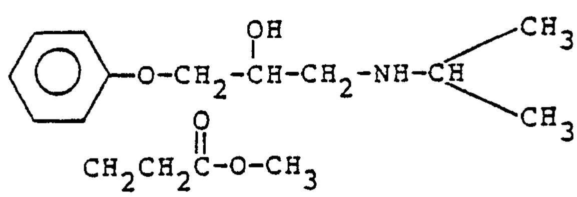

COMPOUND OF EXAMPLE II

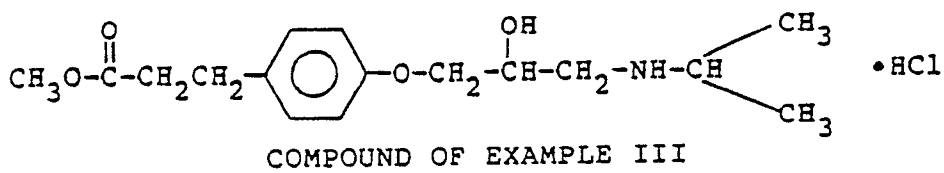

COMPOUND OF EXAMPLE III

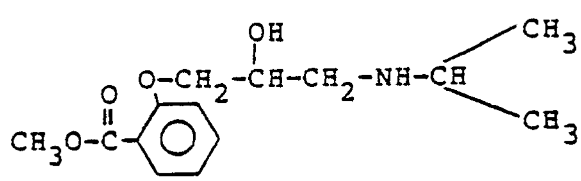

COMPOUND OF EXAMPLE IV

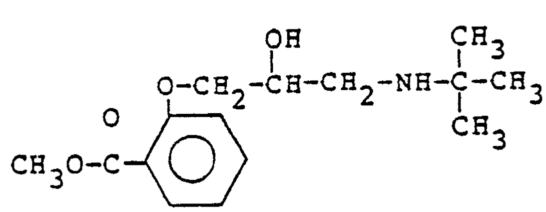

COMPOUND OF EXAMPLE V

All of the compounds tested were synthesized in accordance with the examples of U.S. Patent Application Serial No. 211,345 corresponding to EP—A—53435. Acetonitrile was "HPLC" grade. Distilled water was used to dissolve the compounds and 0.01 N HC1 was used to dissolve compounds requiring an acidic pH for dissolution.

Fresh aqueous humor was collected from eyes of dogs using a 23 gauge needle while fresh dog blood was collected into heparinized Vacutainer® tubes. Fresh liver was homogenized in 0.9% NaCl using a Potter-Elvehjem Teflon pestle and glass honogenizer making a 25% (W/V) homogenate.

A 0.5 ml aliquot of dog aqueous humor, blood, or liver homogenate was incubated with 12.5 µg (0.5 ml) of *beta*-blocker in a Dubnoff shaking metabolic incubator at 37°C for 60 and 120 minutes. Denatured tissue controls were prepared by adding 2.0 ml of acetonitrile into 0.5 ml of aqueous humor, blood, or liver homogenate to destroy esterase activities prior to addition of the *beta*-blockers. These control were then incubated at 37°C for 120 minutes. After 60 and 120 minutes, the incubations were terminated by addition of 2 ml of acetonitrile and immediately mixed using a Vortex® mixer to stop esterase activities.

All samples were centrifuged at 4000 RPM for 10 minutes to sediment denatured proteins. The resultant supernatants were transferred to WISP® vials and analyzed by high pressure liquid chromatography. The hydrolysis of *beta*-blockers in aqueous humor, blood, and liver homogenate was determined by disappearance of the compounds. The extent of enzymatic hydrolysis in each tissue was determined by comparing the amount of each compound (absolute peak area) recovered at each time point to the amount of each compound (absolute peak area) in denatured tissue control and aqueous control samples.

All of the compounds examined were initially tested for chemical hydrolysis in 0.1 N potassium phosphate buffer, pH 7.40, and all were found to be stable for at least three hours (data not shown).

Table 1 summarizes the results of these experiments. The extent of hydrolysis is expressed in terms of the amount of each compound recovered after the incubation period relative to the amount of each compound recovered in the denaturd tissue control. Most of the SABBs were readily hydrolyzed very rapidly (55.5—98.8% in 120 minutes) when incubated with dog blood and liver homogenate. In contrast, all of the compounds tested were resistant to enzymatic hydrolysis by esterases in dog aqueous humor having hydrolysis rates of 0.3—3.6% in 60 minutes and 1.3—13.4% in 120 minutes.

Example VI

The intraocular pressure lowering effect of the compounds of this invention are demonstrated in rabbits with normotensive eyes.

Sterile, isotonic saline solutions of each of the compounds used in procedures of Examples I, II, III, IV, and V are prepared by dissolving 10, 30 and 100 mg samples of each of the active compounds in 1 ml of saline to give 1%, 3% and 10% solutions with pH about 6.0—7.0. Free amines require one equivalent of HCl to effect dissolution.

The intraocular pressure lowering effect of each compound is determined by treating the eyes of healthy rabbits with the above solutions. Three rabbits are used to evaluate the effect of each drug concentration. A standard dose of 50 µl of each drug solution is applied to one eye of each of the three rabbits. Intraocular pressure of both eyes is measured with a pressure tonograph or a Mackay-Marg Tonometer before drug administration and at 15, 30, 45, 60, 120, 180, 240, 300, 360, 420 and 480 min after dosing. Control rabbits are treated similarly with sterile isotonic saline solution. Intraocular pressure lowering in the treated eyes is compared with the untreated eyes, with saline treated eyes and with predrug pressures. All of the test compounds show intraocular pressure-lowering activity.

Example VII

The experiment of Example VI is repeated in all essential details, except that the following compounds are tested:

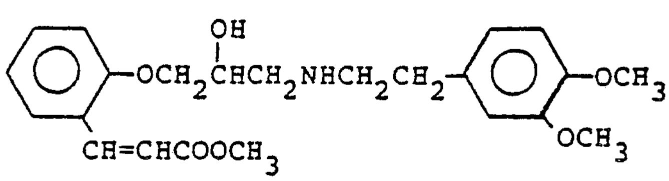

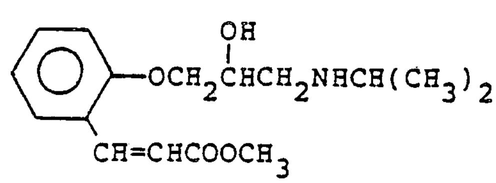

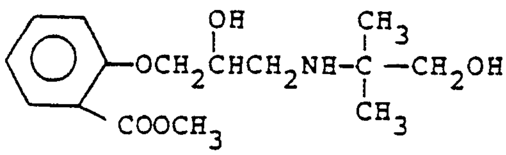

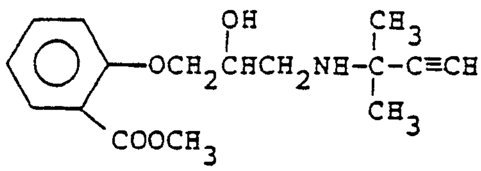

Each of the test compounds exhibit intraocular pressure-lowering activity.

Example VIII

The experiment of Example VI is repeated in all essential details, except that rabbits which have corticosteroid-induced ocular hypertension, as described by Bonomi, L. et al., *Glaucoma*, Eds. R. Pittscrick, A. D. S. Caldwell, Academic Press, New York, pp. 98—107 (1980), are substituted for the normotensive rabbits. Each of the test compounds exhibit intraocular pressure-lowering activity in this model.

TABLE 1

ENZYMATIC HYDROLYSIS OF SABBs BY DOG BLOOD, LIVER HOMOGENATE, AND AQUEOUS HUMOR

| | % HYDROLYZED[1] | | | | | |
|---|---|---|---|---|---|---|
| | BLOOD | | LIVER | | AQUEOUS HUMOR | |
| COMPOUND OF EXAMPLE | 60 min | 120 min | 60 min | 120 min | 60 min | 120 min |
| I | 76.6 | 94.9 | 100 | 100 | 0.3 | 1.3 |
| II | 32.0 | 55.5 | 100 | 100 | 1.5 | 7.3 |
| III | 69.0 | 89.4 | 100 | 100 | 2.4 | 7.5 |
| IV | 65.7 | 88.5 | 11.9 | 40.0 | 3.6 | 8.2 |
| V | 85.6 | 98.8 | 100 | 100 | 3.6 | 13.4 |

[1] Data at each time point are expressed relative to denatured tissue control.

**Claims**

1. Use of a compound of the formula

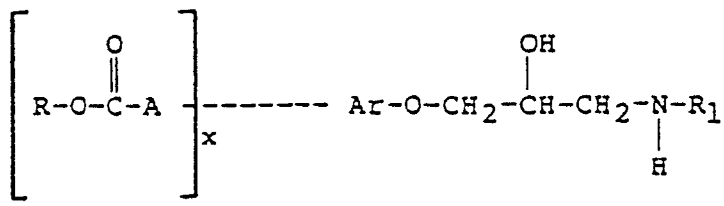

wherein R is alkyl of straight or branched carbon chains from 1 to 10 carbon atoms, cycloalkyl of from 3 to 5 carbon atoms, alkenyl of from 2 to 5 carbon atoms, alkynyl of from 2 to 5 carbon atoms, alkyl carboxymethyl in which the alkyl portion contains from 1 to 5 carbon atoms, aryl carboxymethyl in which the aryl portion contains from 6 to 8 carbon atoms, aryl of from 6 to 10 carbon atoms, or aralkyl wherein the alkyl portion contains from 1 to 5 carbon atoms and the aryl portion represents substituted or unsubstituted monocyclic or polycyclic aromatic of from 6 to 10 carbon atoms; A is a direct bond, alkylene of from 1 to 5 carbon atoms, or alkenylene of from 2 to 5 carbon atoms; x is an integer from 1 to 3, provided that when x is greater than 1, different occurrences of the

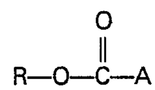

group may be the same or different; Ar is phenyl or naphthyl optionally additionally substituted with $C_1$—$C_{10}$ alkyl, $C_2$—$C_5$ alkenyl, $C_2$—$C_5$ alkynyl, halogen, acetamido, amino, nitro, ($C_1$—$C_{10}$ alkyl) amino, hydroxy, $C_1$—$C_{10}$ hydroxyalkyl, or cyano; $R_1$ is $C_1$—$C_{10}$ alkyl, $C_1$—$C_{10}$ hydroxyalkyl, $C_2$—$C_5$ alkenyl, $C_2$—$C_5$ alkynyl or aralkyl; or a pharmaceutically acceptable salt thereof, for the manufacture of a medicament for the treatment of glaucoma or lowering of intraocular pressure.

2. Use according to claim 1 of a compound wherein A is a direct bond, an alkylene group of from 1 to 5 carbon atoms or alkanylene of from 2 to 5 carbon atoms, and x is 1 or 2.

3. Use according to claim 1 of a compound wherein Ar is phenyl; x is 1 or 2, A is a direct bond, an alkylene group of from 1 to 3 carbon atoms, or alkenylene of from 2 or 3 carbon atoms and at least one of the

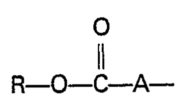

groups is in the ortho position with respect to the

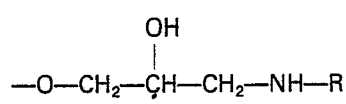

group.

4. Use according to claim 1 of a compound wherein Ar is phenyl, x is 1 or 2; A is alkylene of from 1 to 3 carbon atoms, and at least one of the

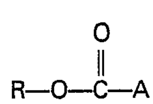

groups is in the para position with respect to the

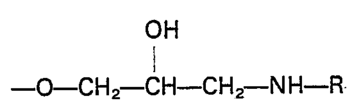

group.

5. Use according to claims 3 or 4 of a compound wherein R is alkyl of from 1 to 5 carbon atoms.

6. Use according to claim 1 of a compound of the formula

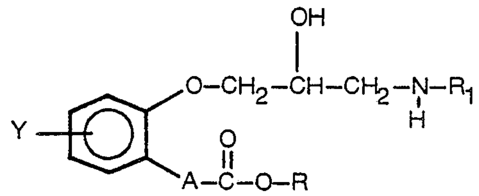

wherein A is an alkylene group of from 1 to 3 carbon atoms, or alkenylene of from 3 to 5 carbon atoms; R is alkyl of from 1 to 5 carbon atoms, alkenyl of from 2 to 5 carbon atoms, or alkynyl of from 3 to 5 carbon atoms; Y is hydrogen, alkyl of from 1 to 5 carbon atoms, alkenyl of from 2 to 5 carbon atoms, alkynyl of from 2 to 5 carbon atoms, alkoxy of from 1 to 5 carbon atoms, halogen, acetamido, amino, nitro, alkylamino of from 1 to 5 carbon atoms, hydroxy, hydroxyalkyl of from 1 to 5 carbon atoms or cyano; and $R_1$ is alkyl of from 1 to 5 carbon atoms, hydroxyalkyl of from 2 to 5 carbon atoms, alkenyl of from 3 to 5 carbon atoms, or aralkyl wherein the alkyl portion contains from 1 to 5 carbon atoms and the aryl portion contains from 6 to 10 carbon atoms.

7. Use according to claim 1 of a compound of the formula

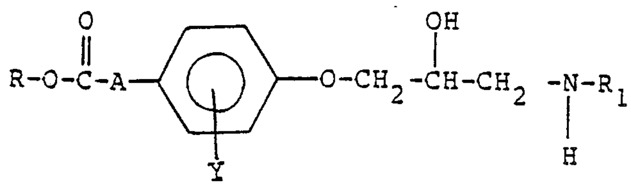

wherein A is alkylene of from 1 to 3 carbon atoms, R is alkyl of from 1 to 5 carbon atoms, or alkynyl of from 3 to 5 carbon atoms; Y is hydrogen, alkyl of from 1 to 5 carbon atoms, alkenyl of from 2 to 5 carbon atoms, alkynyl of from 2 to 5 carbon atoms, alkoxy of from 1 to 5 carbon atoms, halogen, acetamido, amino, nitro, alkylamino of from 1 to 5 carbon atoms, hydroxy, hydroxyalkyl of from 1 to 5 carbon atoms, or cyano; and $R_1$ is alkyl of from 1 to 5 carbon atoms, hydroxyalkyl of from 2 to 5 carbon atoms, alkynyl of from 3 to 5 carbon atoms, or aralkyl wherein the alkyl portion contains from 1 to 5 carbon atoms and the aryl portion contains from 6 to 10 carbon atoms.

8. Use according to claim 1 of a compound of the formula

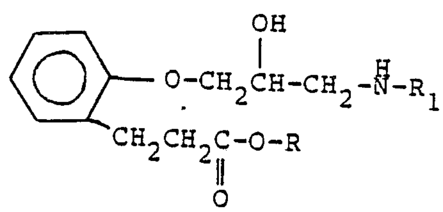

wherein R is methyl, ethyl or propargyl and $R_1$ is isopropyl, t-butyl, hydroxy-t-butyl, dimethyl propargyl, or 3,4-dimethoxyphenethyl.

9. Use according to claim 8 of a compound wherein R is ethyl and $R_1$ is isopropyl or t-butyl.

10. Use according to claim 1 of a compound of the formula

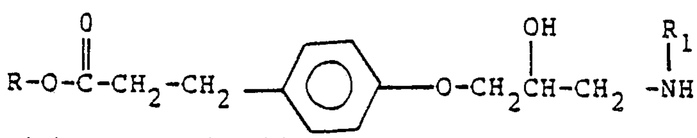

wherein R is methyl, ethyl, or propargyl, and $R_1$ is isopropyl, t-butyl, hydroxy-t-butyl, dimethylpropargyl or 3,4-dimethoxyphenethyl.

11. Use according to claim 10 of a compound wherein R is methyl and $R_1$ is isopropyl or t-butyl.

12. Use according to claim 10 of a compound wherein R is ethyl and $R_1$ is isopropyl or t-butyl.

13. Use according to claim 7 of a compound wherein R is cyclopropylmethyl or propargyl, $R_1$ is isopropyl or t-butyl, Y is hydrogen, and A is ethylene.

14. Use according to claim 1 of a compound of the formula

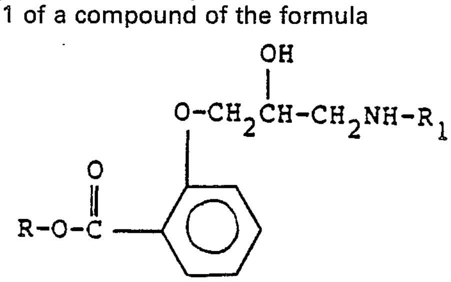

wherein R is methyl, ethyl, or propargyl, $R_1$ is isopropyl, t-butyl, hydroxy-t-butyl, dimethylpropargyl, or 3,4-dimethoxyphenethyl.

15. Use according to claim 1 of a compound of the formula

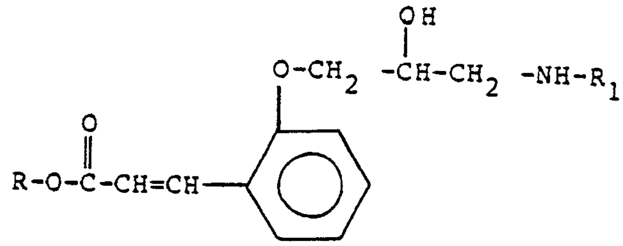

wherein R is methyl, ethyl, or propargyl, $R_1$ is isopropyl, t-butyl, hydroxy-t-butyl, dimethylpropargyl, or 3,4-dimethoxyphenethyl.

**Patentansprüche**

1. Verwendung einer Verbindung der Formel

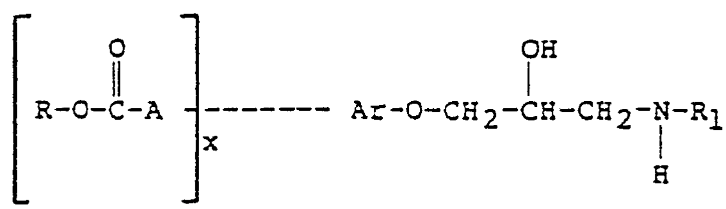

wobei R ein Alkyl aus unverzweigten oder verzweigten Kohlenstoffketten mit 1 bis 10 Kohlenstoffatomen, Cycloalkyl mit 3 bis 5 Kohlenstoffatomen, Alkenyl mit 2 bis 5 Kohlenstoffatomen, Alkinyl mit 2 bis 5 Kohlenstoffatomen, Alkylcarboxymethyl, wobei der Alkylteil 1 bis 5 Kohlenstoffatome enthält, Arylcarboxymethyl, wobei der Arylteil 6 bis 8 Kohlenstoffatome enthält, Aryl mit 6 bis 10 Kohlenstoffatomen oder Aralkyl, wobei der Alkylteil 1 bis 5 Kohlenstoffatome enthält und der Arylteil einen substituierten oder unsubstituierten monozyklischen oder polyzyklischen Aromaten mit 6 bis 10 Kohlenstoffatomen darstellt; A ein direkt gebundenes Alkylen mit 1 bis 5 Kohlenstoffatomen oder Alkenylen mit 2 bis 5 Kohlenstoffatomen; x eine ganze Zahl von 1 bis 3, vorausgesetzt, daß, wenn x größer als 1 ist, das Auftreten der

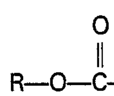-Gruppe

an verschiedenen Stellen gleich oder verschieden sein kann; Ar Phenyl oder Naphthyl, wahlweise zusätzlich substituiert mit $C_1$—$C_{10}$-Alkyl, $C_2$—$C_5$-Alkenyl, $C_2$—$C_5$-Alkinyl, Halogen, Acetamido, Amino, Nitro, ($C_1$—$C_{10}$-Alkyl)-amino, Hydroxy, $C_1$—$C_{10}$-Hydroxyalkyl oder Cyano; und $R_1$ $C_1$—$C_{10}$-Alkyl, $C_1$—$C_{10}$-Hydroxyalkyl, $C_2$—$C_5$-Alkenyl, $C_2$—$C_5$-Alkinyl oder Aralkyl ist; oder eine pharmazeutisch akzeptables Salz derselben zur Herstellung eines Medikaments zur Behandlung von Glaukom oder zum Absenken des Augeninnendrucks.

2. Verwendung nach Anspruch 1 von einer Verbindung, bei der A eine direkt gebundene Alkylengruppe mit 1 bis 5 Kohlenstoffatomen oder eine Alkenylengruppe mit 2 bis 5 Kohlenstoffatomen und x 1 oder 2 ist.

3. Verwendung nach Anspruch 1 von einer Verbindung, bei der Ar Phenyl; x 1 oder 2; A eine direkt gebundene Alkylengruppe mit 1 bis 3 Kohlenstoffatomen oder eine Alkenylengruppe mit 2 bis 3 Kohlenstoffatomen und wenigstens eine der

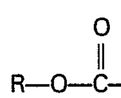-Gruppen

in der ortho-Position hinsichtlich der

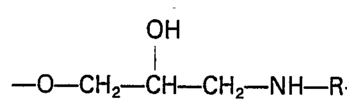-Gruppe

ist.

4. Verwendung nach Anspruch 1 von einer Verbindung, bei der Ar Phenyl; x 1 oder 2; A Alkylen mit 1 bis 3 Kohlenstoffatomen und wenigstens eine der

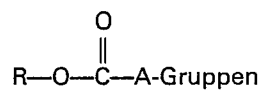

in der para-Position hinsichtlich der

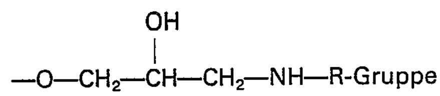

ist.

5. Verwendung nach Anspruch 3 oder 4 von einer Verbindung, bei der R Alkyl mit 1 bis 5 Kohlenstoffatomen ist.

6. Verwendung nach Anspruch 1 von einer Verbindung der Formel

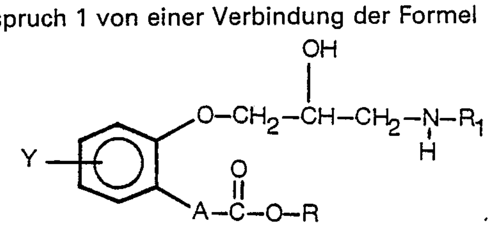

wobei A ein Alkylengruppe mit 1 bis 3 Kohlenstoffatomen oder eine Alkenylengruppe mit 3 bis 5 Kohlenstoffatomen; R Alkyl mit 1 bis 5 Kohlenstoffatomen, Alkenyl mit 2 bis 5 Kohlenstoffatomen oder Alkinyl mit 3 bis 5 Kohlenstoffatomen; Y Wasserstoff, Alkyl mit 1 bis 5 Kohlenstoffatomen, Alkenyl mit 2 bis 5 Kohlenstoffatomen, Alkinyl mit 2 bis 5 Kohlenstoffatomen, Alkoxy mit 1 bis 5 Kohlenstoffatomen, Halogen, Acetamido, Amino, Nitro, Alkylamino mit 1 bis 5 Kohlenstoffatomen, Hydroxy, Hydroxyalkyl mit 1 bis 5 Kohlenstoffatomen oder Cyano und $R_1$ Alkyl mit 1 bis 5 Kohlenstoffatomen, Hydroxyalkyl mit 2 bis 5 Kohlenstoffatomen, Alkenyl mit 3 bis 5 Kohlenstoffatomen oder Aralkyl wobei der Alkylteil 1 bis 5 Kohlenstoffatome und der Arylteil 6 bis 10 Kohlenstoffatome enthält, ist.

7. Verwendung nach Anspruch 1 von einer Verbindung der Formel

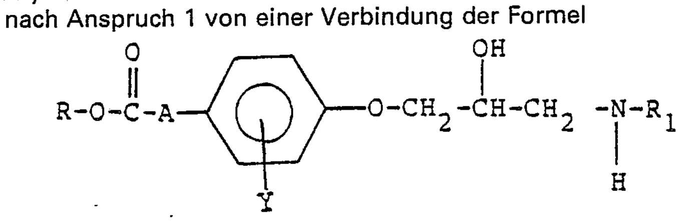

wobei A Alkylen mit 1 bis 3 Kohlenstoffatomen; R Alkyl mit 1 bis 5 Kohlenstoffatomen oder Alkinyl mit 3 bis 5 Kohlenstoffatomen; Y Wasserstoff, Alkyl mit 1 bis 5 Kohlenstoffatomen, Alkenyl mit 2 bis 5 Kohlenstoffatomen, Alkinyl mit 2 bis 5 Kohlenstoffatomen, Alkoxy mit 1 bis 5 Kohlenstoffatomen, Halogen, Acetamido, Amino, Nitro, Alkylamino mit 1 bis 5 Kohlenstoffatomen, Hydroxy, Hydroxyalkyl mit 1 bis 5 Kohlenstoffatomen, oder Cyano; und $R_1$ Alkyl mit 1 bis 5 Kohlenstoffatomen, Hydroxyalkyl mit 2 bis 5 Kohlenstoffatomen, Alkinyl mit 3 bis 5 Kohlenstoffatomen oder Aralkyl, wobei der Alkylteil 1 bis 5 Kohlenstoffatome und der Arylteil 6 bis 10 Kohlenstoffatome enthält, ist.

8. Verwendung nach Anspruch 1 von einer Verbindung der Formel

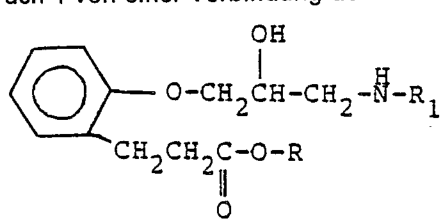

wobei R Methyl, Ethyl oder Propargyl und $R_1$ Isopropyl, t-Butyl, Hydroxy-t-butyl, Dimethylpropargyl oder 3,4-Dimethoxyphenethyl ist.

9. Verwendung nach Anspruch 8 von einer Verbindung, bei der R Ethyl und $R_1$ Isopropyl oder t-Butyl ist.

10. Verwendung nach Anspruch 1 von einer Verbindung der Formel

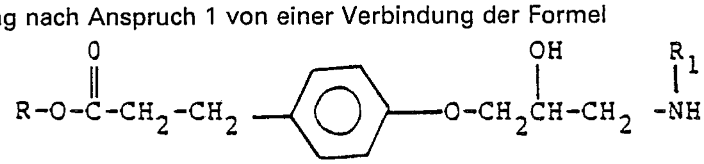

wobei R Methyl, Ethyl oder Propargyl und $R_1$ Isopropyl, t-Butyl, Hydroxy-t-butyl, Dimethylpropargyl oder 3,4-Dimethoxyphenethyl ist.

11. Verwendung nach Anspruch 10 von einer Verbindung, bei der R Methyl und $R_1$ Isopropyl oder t-Butyl ist.

12. Verwendung nach Anspruch 10 von einer Verbindung, bei der R Ethyl und $R_1$ Isopropyl oder t-Butyl ist.

13. Verwendung nach Anspruch 7 von einer Verbindung, bei der R Cyklopropylmethyl oder Propargyl, $R_1$ Isopropyl oder t-Butyl, Y Wasserstoff und A Ethylen ist.

14. Verwendung nach Anspruch 1 von einer Verbindung der Formel

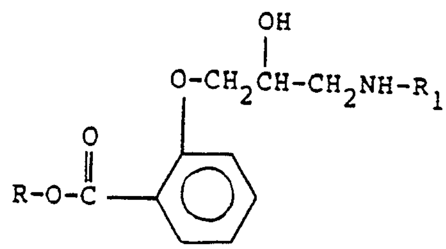

wobei R Methyl, Ethyl oder Propargyl, $R_1$ Isopropyl, t-Butyl, Hydroxy-t-butyl, Dimethylpropargyl oder 3,4-Dimethoxyphenetyl ist.

15. Verwendung nach Anspruch 1 von einer Verbindung der Formel

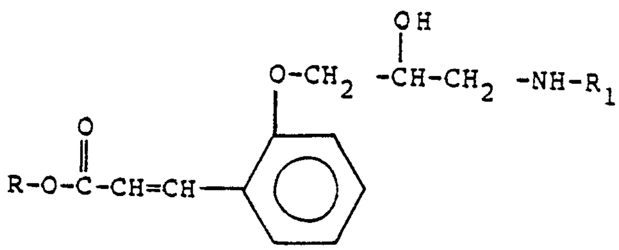

wobei R Methyl, Ethyl oder Propargyl, $R_1$ Isopropyl, t-Butyl, Hydroxy-t-butyl, Dimethylpropargyl oder 3,4-Dimethoxyphenetyl ist.

**Revendications**

1. Utilisation d'un composé de formule

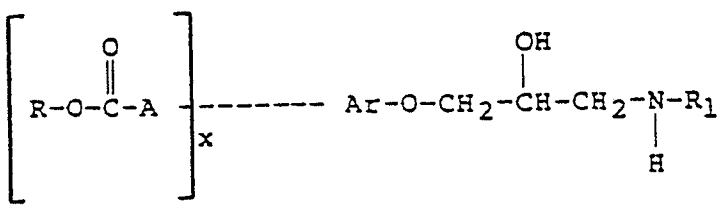

dans laquelle R est un radical alkyle à chaîne droite ou ramifiée de 1 à 10 atomes de carbone, cycloalkyle de 3 à 5 atomes de carbone, alcényle de 2 à 5 atomes de carbone, alcynyle de 2 à 5 atomes de carbone, alkyl-carboxyméthyle dont la partie alkyle contient de 1 à 5 atomes de carbone, aryl-carboxyméthyle dont la partie aryle contient de 6 à 8 atomes de carbone, aryle de 6 à 10 atomes de carbone, ou aralkyle dont la partie alkyle contient de 1 à 5 atomes de carbone et dont la partie aryle représente un radical aromatique monocyclique ou polycyclique, substitué ou non substitué, de 6 à 10 atomes de carbone; A est une liaison directe où un radical alkylène de 1 à 5 atomes de carbone, ou alcénylène de 2 à 5 atomes de carbone; x est un nombre entier de 1 à 3, à condition que, quand x est plus grand que 1, les différents groupements

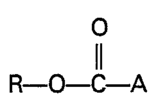

puissent être identiques ou différents; Ar est un radical phényle ou naphtyle, facultativement substitué additionellement par alkyle en $C_1$—$C_{10}$, alcényle en $C_2$—$C_5$, alcynyle e $C_2$—$C_5$, halogène, acétamide, amine, nitro, (alkyl en $C_1$—$C_{10}$) amine, hydroxy, hydroxyalkyle en $C_1$—$C_{10}$, ou cyano; $R_1$ est un radical alkyle en $C_1$—$C_{10}$, hydroxyalkyle en $C_1$—$C_{10}$, alcényle en $C_2$—$C_5$, alcynyle en $C_2$—$C_5$, ou aralkyle; ou bien d'un sel pharmaceutiquement acceptable de ce composé, pour la fabrication d'un médicament destiné au traitement du glaucome ou à l'abaissement de la pression intra-oculaire.

2. Utilisation, selon la revendication 1, d'un composé dans lequel A est une liaison directe ou un radical alkylène de 1 à 5 atomes de carbone ou alcénylène de 2 à 5 atomes de carbone, et x est égal à 1 ou 2.

3. Utilisation, selon la revendication 1, d'un composé dans lequel Ar est un radical phényle, x est égal à 1 ou 2, A est une liaison directe ou un radical alkylène de 1 à 3 atomes de carbone, ou alcénylène de 2 ou 3 atomes de carbone, et au moins un groupement

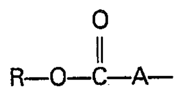

est en position ortho par rapport au groupement

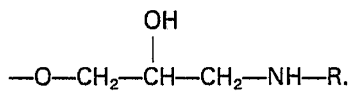

4. Utilisation, selon la revendication 1, d'un composé dans lequel Ar est un radical phényle, x est égal à 1 ou 2, A est un radical alkylène de 1 à 3 atomes de carbone, et au moins un des groupements

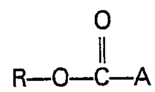

est en position para par rapport au groupement

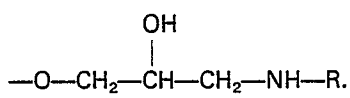

5. Utilisation, selon la revendication 3 ou 4, d'un composé dans lequel R est un radical alkyle de 1 à 5 atomes de carbone.

6. Utilisation, selon la revendication 1, d'un composé de formule

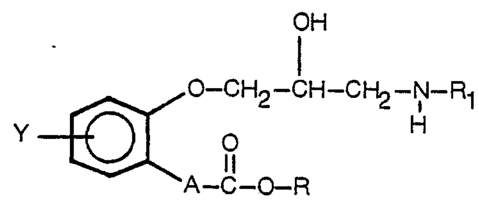

dans laquelle A est un radical alkylène de 1 à 3 atomes de carbone, ou alcénylène de 3 à 5 atomes de carbone; R est un radical alkyle de 1 à 5 atomes de carbone, alcényle de 2 à 5 atomes de carbone ou alcynyle de 3 à 5 atomes de carbone; Y est l'hydrogène, un radical alkyle de 1 à 5 atomes de carbone, alcényle de 2 à 5 atomes de carbone, alcynyle de 2 à 5 atomes de carbone, alcoxy de 1 à 5 atomes de carbone, un halogène, un groupement acétamide, amine, nitro, alkylamine de 1 à 5 atomes de carbone, hydroxy, hydroxyalkyle de 1 à 5 atomes de carbone, ou cyano; et $R_1$ est un radical alkyle de 1 à 5 atomes de carbone, hydroxyalkyle de 2 à 5 atomes de carbone, alcényle de 3 à 5 atomes de carbone, ou aralkyle dont la partie alkyle contient de 1 à 5 atomes de carbone et dont la partie aryle contient de 6 à 10 atomes de carbone.

7. Utilisation, selon la revendication 1, d'un composé de formule

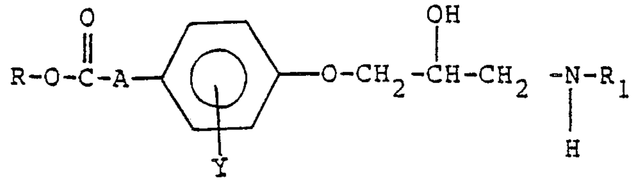

dans laquelle A est un radical alkylène de 1 à 3 atomes de carbone, R est un radical alkyle de 1 à 5 atomes de carbone ou alcynyle de 3 à 5 atomes de carbone; Y est l'hydrogène, un radical alkyle de 1 à 5 atomes de carbone, alcényle de 2 à 5 atomes de carbone, alcynyle de 2 à 5 atomes de carbone, alcoxy de 1 à 5 atomes de carbone, un halogène, un groupement acétamide, amine, nitro, alkylamine de 1 à 5 atomes de carbone, hydroxy, hydroxyalkyle de 1 à 5 atomes de carbone, ou cyano; et $R_1$ est un radical alkyle de 1 à 5 atomes de carbone, hydroxyalkyle de 2 à 5 atomes de carbone, alcynyle de 3 à 5 atomes de carbone, ou aralkyle dont la partie alkyle contient de 1 à 5 atomes de carbone et dont la partie aryle contient de 6 à 10 atomes de carbone.

8. Utilisation, selon la revendication 1, d'un composé de formule

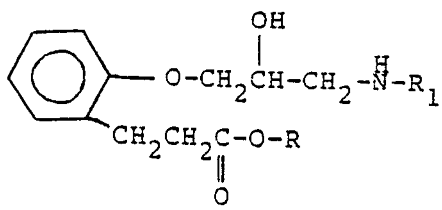

dans laquelle R est un radical méthyle, éthyle ou propargyle et $R_1$ est un radical isopropyle, t-butyle, hydroxy-t-butyle, diméthyl propargyle, ou 3,4-diméthoxyphénéthyle.

9. Utilisation, selon la revendication 8, d'un composé dans lequel R est un radical éthyle et $R_1$ est un radical isopropyle ou t-butyle.

10. Utilisation, selon la revendication 1, d'un composé de formule:

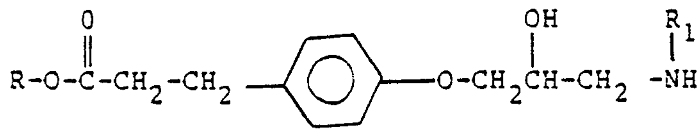

dans laquelle R est un radical méthyle, éthyle ou propargyle, et $R_1$ est un radical isopropyle, t-butyle, hydroxy-t-butyle, diméthylpropargyle, ou 3,4-diméthoxyphénéthyle.

11. Utilisation, selon la revendication 10, d'un composé dans lequel R est le radical méthyle et $R_1$ est un radical isopropyle ou t-butyle.

12. Utilisation, selon la revendication 10, d'un composé dans lequel R est le radical éthyle et $R_1$ est le radical isopropyle ou t-butyle.

13. Utilisation, selon la revendication 7, d'un composé dans lequel R est un radical cyclopropylméthyle ou propargyle, $R_1$ est un radical isopropyle ou t-butyle, Y est l'hydrogène et A est le groupement éthylène.

14. Utilisation, selon la revendication 1, d'un composé de formule

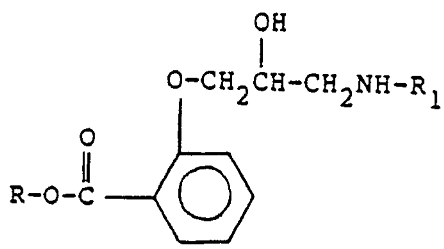

dans laquelle R est un radical méthyle, éthyle, ou propargyle, $R_1$ est un radical isopropyle, t-butyle, hydroxy-t-butyle, diméthylpropargyle, ou 3,4-diméthoxyphénéthyle.

15. Utilisation, selon la revendication 1, d'un composé de formule

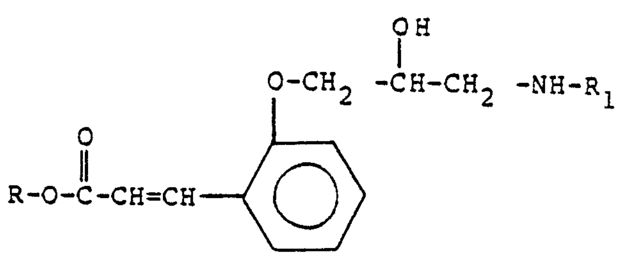

dans laquelle R est un radical méthyle, éthyle, ou propargyle et $R_1$ est un radical isopropyle, t-butyle, hydroxy-t-butyle, diméthylpropargyle, ou 3,4-diméthoxyphénéthyle.